# FASCICULE DE BREVET EUROPEEN

(11) **EP 2 445 477 B1**
(45) Date de publication et mention de la délivrance du brevet: **11.02.2015**
(21) Numéro de dépôt: 10728817.7
(22) Date de dépôt: 25.05.2010
(51) Int. Cl.: A61K 8/44, A61K 8/81, A61Q 19/00, C08K 5/20, C08L 25/08, C08L 33/02, C08L 47/00

(54) **COMPOSITION COSMETIQUE A BASE DE RESINES ECHANGEUSES D'IONS CHARGEES AVEC LES LIPOAMINOACIDES**
KOSMETISCHE ZUSAMMENSETZUNG AUS MIT LIPOAMINSÄUREN GEFÜLLTEN IONENAUSTAUSCHHARZEN
COSMETIC COMPOSITION MADE FROM ION-EXCHANGE RESINS FILLED WITH LIPOAMINOACIDS

(30) Priorité: 24.06.2009 FR 0954317
(43) Date de publication de la demande: 02.05.2012
(73) Titulaire: Societe D'Exploitation De Produits Pour Les Industries Chimiques Seppic, 75007 Paris (FR)
(72) Inventeur: DUCCINI, Yves, F-81570 Vielmur/Agout (FR)
(74) Mandataire: Grout de Beaufort, François-Xavier
(86) Numéro de dépôt international: PCT/FR2010/051007
(87) Numéro de publication internationale: WO 2010/149889

(56) Documents cités:
- EP-A2- 1 250 937
- GB-A- 885 087

## Description

La présente invention concerne des compositions à base de résines échangeuses d'ions chargées avec des lipoaminoacides, des formulations cosmétiques comprenant de telles compositions ainsi qu'un procédé de préparation de ces compositions.

De nombreuses molécules biologiquement actives à la surface de la peau sont très difficiles à formuler pour préparer des compositions cosmétiques les comprenant et la mise au point de formes galéniques comprenant ces molécules biologiquement actives pose de gros problèmes au formulateur.

La demande de brevet EP11175914A2 divulgue une composition de «résinate» qui comprend une résine échangeuse d'ions chargée d'une substance active pharmaceutique (telle que la lansoprazole, la nifedipine, la cisapride, la nelfinavir). La demande de brevet EP1396265A1 divulgue une composition pharmaceutique qui comprend de 5% à 75% d'un ingrédient actif qui cause une irritation gastrointestinale et de 25% à 95% d'une résine échangeuse d'ions ; ledit ingrédient actif étant un acide polyphosphonique.

La demande de brevet EP1346732A2 revendique une forme dosée qui comprend un « résinate » d'un agent actif hygroscopique, c'est à dire un agent hygroscopique comme l'acide valproique, des sels de choline, d'acide pamidronique, échangé sur une résine échangeuse d'ions.

Ces demandes ne divulguent pas d'applications topiques de compositions comprenant des résines échangeuses d'ions et de composés biologiquement actifs.

Il est également très difficile de contrôler la vitesse de libération de ces molécules biologiquement actives lorsqu'elles sont appliquées sur la peau au travers de crèmes, lotions, gels ou d'émulsions. En général lors de l'application de telles formulations, les molécules biologiquement actives sont libérées en une seule fois. Parmi les molécules posant problème, on peut citer de manière non exhaustive les molécules suivantes la caféine utilisée comme molécule amincissante et possédant une activité lipolytique, la DiMéthylEthanolAmine (ou DMEA) utilisée omme agent antiride, les vitamines E et C utilisées comme agents antiradicalaires, des extraits de plantes d'algues et de fruits utilisés pour leurs vertus réparatrices ou protectrices sur la peau et enfin des lipoaminoacides utilisés par exemple comme agents actifs éclaircissants de la peau, comme agents actifs apaisants, comme agent actifs amincissants et lipolytiques.

Les difficultés de formulation galénique de ces molécules sont de tous ordres.

Certaines molécules sont liquides, mais insolubles ou faiblement solubles dans le milieu cosmétique utilisé comme vecteur, d'autres sont pâteuses à température ambiante, d'autres se délitent à température ambiante, d'autres sont hygroscopiques ou se détruisent en présence de lumière.

Nombre de ces molécules procurent une odeur forte dans les compositions les comprenant, voire désagréable et il est très difficile pour les formulateurs de cosmétiques de les incorporer dans des crèmes ou des lotions, sans que le consommateur ne perçoive cette odeur.

Enfin pour obtenir une efficacité maximale, les formulateurs préféreraient que les molécules actives se libèrent de façon prolongée sur la peau de façon à présenter une activité uniforme dans le temps et de longue durée. Au contraire, elles se libèrent dès l'application de la composition cosmétique, pouvant se présenter sous toute forme comme par exemple un gel ou une crème, et les quantités libérées sont souvent trop importantes par rapport à l'effet recherché mais leur activité est de courte durée.

De nombreuses solutions, comme par exemple celles mettant en oeuvre des technologies d'encapsulation ou d'absorption sur un support inerte ont été développées par les hommes de l'art pour résoudre les problèmes préalablement décrits.

Nulle n'est totalement satisfaisante et surtout nulle ne résout à la fois les problèmes d'odeurs, de formulation et de libération contrôlée.

Parmi ces solutions on peut citer : l'absorption sur des supports poreux tels que la silice, les zéolites, les celluloses et ses dérivés, les polyméthylméthacrylates, les polyméthacrylates ou les polyacrylates. Les inconvénients majeurs de l'absorption sur support poreux sont : la faible quantité de molécules actives absorbées, la perception par le consommateur de la présence de ces supports poreux du fait de leurs granulométries, l'impossibilité d'absorber certains actifs, et la quasi impossibilité de contrôler la libération des molécules actives. En outre, l'odeur est souvent encore perceptible. Il existe également la possibilité de mettre en oeuvre des technologies d'encapsulation par coacervation ou par coacervation complexe, comme par exemple pour le menthol pour son incorporation dans les dentifrices. Outre son coût très élevé, ces technologies ne sont pas utilisables pour des molécules hydrophiles et la vitesse de libération est brutale, lors de la rupture de la coque du coacervat.

La technologie d'immobilisation dans des capsules de cires nécessite de chauffer les molécules et leur libération est conditionnée par une élévation de température ou une forte abrasion. De plus, cette technologie n'est pas adaptée pour des molécules biologiquement actives sensibles à la chaleur qui peuvent alors se dégrader et générer l'apparition de sous-produits de dégradation lors du phénomène de libération par élévation de température.

Les techniques de polymérisation inter faciales mettent en jeu des molécules souvent assez toxiques et sont réservées, du fait de leur coût, à des applications à haute valeur ajoutée.

Enfin, toutes les technologies basées sur de la thermodynamique de tensioactifs (tels que par exemple les technologies de préparation des liposomes ou des sphérulites) sont des technologies difficiles à mettre en oeuvre, souvent peu fiables lorsqu'il s'agit de savoir exactement combien de molécules actives sont protégées, combien sont libres. De plus leur stabilité dans le temps est très aléatoire.

Un but de la présente invention est de pallier tout ou partie des inconvénients de l'art antérieur relevés ci-dessus.

A cette fin, la présente invention a pour objet une composition (C) de résines échangeuses d'ions à base de copolymère styrène - divinylbenzène ou de copolymère acrylique - divinylbenzène chargées avec au moins un lipoaminoacide de formule (I) : 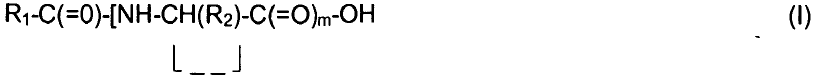 dans laquelle R₁ représente la chaîne caractérisante d'un acide gras, saturé ou insaturé, linéaire ou ramifié, comportant de 7 à 21 atomes de carbone, R₂ représente la chaîne caractérisante d'un acide aminé et m est compris entre 1 et 50, ou d'un mélange desdits composés de formule (I).

Selon un aspect préféré de l'invention, la composition (C) définie ci-dessus est caractérisée en ce que ladite résine contient au moins une fonction ammonium quaternaire.

Selon un aspect préféré de l'invention, la composition (C) définie ci-dessus est caractérisée en ce que ledit lipoaminoacide est choisi parmi : le N-(ω-undecylenoyl) phenylalanine, l'octanoyl glycine, l'undecylenoyl glycine, le palmitoyl proline, le dipalmitoylhydroxyproline, le cocoylalanine, le palmitoylglycine.

Par résine échangeuse d'ion chargée avec un lipoaminoacide, on désigne une résine dont l'ion échangeable est remplacé par ledit lipoaminoacide sous sa forme saline.

Dans la formule (I), R₁-C(=O)- représente notamment l'un des radicaux octanoyle, décanoyle, undécylènoyle, dodécanoyle, tétradécanoyle, hexadécanoyle, octadécanoyle, eicosanoyle, docosanoyle, 8-octadécènoyle, éicosènoyle, 13-docosènoyle, 9,12-octadécadiènoyle ou 9,12,15-octaclécatriénoyle.

Plus particulièrement, le fragment R₁-C(=O) est choisi parmi les radicaux octanoyle, w-undécylènoyle, dodécanoyle, hexadécanoyle, 8-octadécènoyle, 13-docasènoyle, 9,12-octadécadiènoyle ou 9,12,15-octadécatriénoyle.

Pour un acide aminé représenté par la formule générale IIIa) :

H₂N-CH(R₂)-C(=O)-OH (IIIa),

comme pour un acide aminé cyclique représenté par la formule (IIIb) : 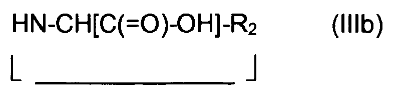 la chaîne caractérisante sera la chaîne représentée par R₂.

R₂ représente notamment la chaîne caractérisante d'un acide aminé choisi parmi la glycine, l'alanine, la sérine, l'acide aspartique, l'acide glutamique, la valine, la thréonine, l'arginine, la lysine, la proline, la leucine, la phénylalanine, l'isoleucine, l'histidine, la tyrosine, le tryptophane, l'asparagine, la glutamine, la cystéine, la cystine, la méthionine, l'hydroxy proline, l'hydroxy lysine, la sarcosine ou l'omithine.

L'invention a principalement pour objet, une composition telle que définie ci-dessus dans laquelle ledit composé de formule (I) telle que définie précédemment, a au moins un des restes

-HN-CH(R₂)-C(=O)- (IIIa),

ou 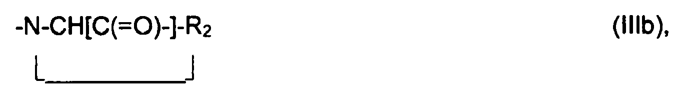 dans lequel, R₂ représente la chaîne caractérisante de la phénylalanine, de la tyrosine, de l'histidine, de la méthionine de la cystéine ou du tryptophane.

L'invention a plus particulièrement pour objet, une composition comprenant un composé de formule (I), telle que définie précédemment dans laquelle m est un nombre décimal compris entre 1 et 10 et est de préférence inférieur à 5.

Selon un aspect tout particulier de la présente invention, dans la formule (I) telle que définie précédemment, m est inférieur ou égal à 2 et est plus particulièrement inférieur ou égal à 1,4.

Selon un autre aspect tout particulier de la présente invention, dans la formule (I) telle que définie précédemment, m égal à 1.

Selon une autre variante particulière de la présente invention, ladite composition comprend un mélange de composés de formule (I) telle que définie précédemment et, plus particulièrement,
ou bien un mélange de composés de formules (I) comportant toutes le même fragment R₁-C(=O),
ou bien un mélange de composés de formules (I) dans lesquelles m est égal à 1 et comportant toutes le même fragment

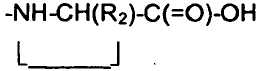

La présente invention a également pour objet une formulation cosmétique comprenant la composition (C) telle que définie ci-dessus, caractérisée en ce que le pH de ladite formulation est compris entre 5 et 7 et la proportion massique de ladite composition est inférieure à 1,5% de la masse totale.

Selon un mode particulier de l'invention, la formulation telle que définie précédemment est caractérisée en ce que la proportion massique de la composition (C) telle que définie ci-dessus est comprise entre 0,5% et 0,8% de la masse totale.

Selon un mode particulier de l'invention, la formulation telle que définie précédemment est caractérisée en ce qu'elle se présente sous la forme d'un gel crème, d'une émulsion, d'une poudre, d'une dispersion aqueuse, d'une dispersion huileuse.

L'invention a également pour objet l'utilisation d'une formulation telle que définie ci-dessus pour une application cosmétique sur la peau humaine.

La formulation telle que définie ci-dessus peut également être utilisée comme antiride en étant caractérisée en ce que le lipoaminoacide employé est choisi parmi : le dipalmitoylhydroxy proline, le cocoylalanine, le palmitoylglycine.

La formulation telle que définie ci-dessus peut également être utilisée comme agent d'éclaircissement de la peau ou agent amincissant en étant caractérisée en ce que le lipoaminoacide employé est choisi parmi : le N-(w-undecylenoyl) phenylalanine, le palmitoyl proline.

L'invention a également pour objet un procédé de préparation d'une composition (C) telle que définie ci-dessus, comprenant l'étape :
a) mise en contact d'une résine échangeuse d'ions à base de copolymère styrène - divinylbenzène ou styrène - acrylique. contenant au moins un ammonium quaternaire avec une solution de lipoaminoacide de formule (I).

Selon un des ses caractéristiques, le procédé tel que défini précédemment est caractérisé en ce que l'étape a) est une étape de percolation où un lit de ladite résine est traversée par ladite solution de lipoaminoacide à charger.

Les résines échangeuses d'ions sont des macromolécules insolubles portant des groupements ioniques qui ont la propriété d'échanger de façon réversible certains de leurs ions, au contact d'autres ions provenant d'un milieu différent et distinct.

Ces résines possèdent une certaine capacité de rétention des ions (exprimée par gramme de résine sèche), qui correspond au nombre de millimoles (mmol) d'ions que la résine peut échanger par unité de masse de ladite résine. Les résines échangeuses d'ions sont en outre caractérisées par leurs tailles de particules et par le pK de leur groupe fonctionnel.

Plus particulièrement, les résines échangeuses d'anions, encore nommées résines anioniques ou résines basiques, portent des groupes fonctionnels chargés positivement et ont la propriété d'échanger de façon réversible certains de leurs anions associés aux groupements fonctionnels chargés positivement, au contact d'autres anions tels que Cl⁻, OH⁻, SO₄²⁻, etc, compris dans le milieu distinct mis alors au contact de ladite résine.

De manière analogue, les résines échangeuses de cations, encore nommées résines cationiques ou résines acides, portent des groupes fonctionnels chargés négativement et ont la propriété d'échanger de façon réversible certains de leurs cations associés aux groupements fonctionnels chargés négativement, au contact d'autres cations tels que Na⁺, H⁺, Ca⁺, etc, compris dans le milieu distinct mis alors au contact de ladite résine.

Les résines cationiques renfermant des unités monomériques de styrène et de divinylbenzène ou de dérivés acryliques, possèdent des groupements fonctionnels portant des fonctions sulfoniques, caractérisant des résines cationiques fortes (par exemple l'Amberlite® IRP88, l'Amberlite® IRP69, Dowex®, etc.), ou des groupements fonctionnels portant des fonctions carboxyliques, caractérisant des résines cationiques faibles (par exemple l'AmberliteIRP64). Dans un mode de réalisation préféré, la résine organique échangeuse d'ions est une résine organique anionique possédant une matrice styrène-divinyibenzène telle que celles commercialisées sous la marque Amberlite® 1RA4004 Chloride par la société Rohm & Haas, sous la marque Dowex® 2*8 Chloride par la société Dow Chemical Co., sous la marque Dowex 1*8 Chloride par la société Dow Chemical Co. et sous la marque Dowex 1*2 Chloride par la société Dow Chemical Co.

Selon un autre mode de réalisation préféré, la résine organique échangeuse d'ions est une résine organique échangeuses d'anions comprenant des unités monomériques de dérivés de l'acide acrylique, telle que celle commercialisée sous la marque Amberlite® IRA67 par la société Rohm & Haas.

Selon un mode de réalisation encore plus préféré, la résine organique échangeuse d'anions est une résine comprenant un copolymère renfermant des unités monomériques de styrène et de divinylbenzène et comportant des fonctions ammonium quaternaire, également nommée cholestyramine et commercialisée notamment sous la marque Duolite® AP143 par la société Rohm & Haas. L'anion échangeable de cette résine est l'anion chlorure.

Selon un autre mode de réalisation préféré, la résine organique échangeuse d'ions est une résine organique cationique comprenant un copolymère renfermant des unités monomériques de styrène et de divinylbenzène comme par exemple la résine commercialisée sous la marque Amberlite® IRP69 ou une résine comprenant un copolymère renfermant des unités monomériques d'acide acrylique et de divinylbenzène comme par exemple la résine commercialisée sous la marque Amberlite® IRP64.

Dans un autre mode de réalisation préféré, une résine organique anionique telle que décrite ci-dessus, par exemple la résine Duolite® AP143 est utilisée en combinaison avec une résine organique échangeuse de cations telle que décrite ci-dessus, par exemple l'Amberlite®.

De façon générale, la composition objet de l'invention comprenant des composés de formule (I) et des résines échangeuses d'ions, est associée à de nombreux types d'ingrédients chimiques habituellement utilisés dans les formulations cosmétiques, qu'il s'agisse, de corps gras, de solvants organiques, d'épaississants, de gélifiants, d'adoucissants, d'antioxydants, d'opacifiants, de stabilisants, de moussants, de parfums, d'émulsionnants, ioniques ou non, de charges, de séquestrants, de chélateurs, de conservateurs, de filtres chimiques ou de filtres minéraux, d'huiles essentielles, de matières colorantes, de pigments, d'actifs hydrophiles ou lipophiles, d'humectants, comme par exemple la glycérine, de conservateurs, de colorants, de parfums, d'actifs cosmétiques, de filtres solaires minéraux ou organiques, de charges minérales comme les oxydes de fer, oxydes de titane et le talc, de charges synthétiques comme les nylons et les polyméthacrylate de méthyle) réticulés ou non, d'élastomères silicone, de séricites ou d'extraits de plantes ou encore de vésicules lipidiques ou tout autre ingrédient habituellement utilisé en cosmétique.

Comme exemples d'huiles que l'on peut associer à la composition objet de l'invention, on peut citer, les paraffines, les isoparaffines, les huiles blanches minérales, les huiles végétales, les huiles animales, les huiles de synthèse, les huiles siliconées et les huiles fluorées ; et plus particulièrement :
- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales éthoxylées ;
- les huiles d'origine animale, telles que le squalène, le squalane ;
- les huiles minérales, telles que l'huile de paraffine, l'huile de vaseline et les isoparaffines ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycéricles, diglycérides et triglycérides d'acides gras comme le
triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles.

Comme autre matière grasse que l'on peut associer à la composition objet de l'invention, on peut citer les alcools gras ou les acides gras.

Parmi les polymères épaississants et/ou émulsionnants que l'on peut associer à la composition objet de la présente invention, il y a par exemple :
- les polymères de type polyélectrolytes comme par exemple les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino]1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atonies de carbone. De tels polymères sont commercialisés respectivement sous les appellations SIMULGEL™ EG, SEPIGEL™ 305, SIMULGEL™ NS, SIMULGEL™ 15 800, SIMULGEL™ A, SIMULGEL™ EPG, SIMULGEL™ INS100, SIMULGEL™ FL, SIMULGEL SMS 88, SEPIGEL™ 501, SEPIGEL™ 502, SEPIPLUS™ 250, SEPIPLUS™ 265, SEPIPLUS™ 400, SEPIPLUS S, SEPINOV™ EMT 10, SEPINOV P88 CARBOPOL™, ULTREZ™ 10, ACULYN™ PEMULEN™ TR1, PEMULEN™ TR2, LUVIGEL™ EM, SALCARE™ SC91, SALCARE™ SC92, SALCARE™ SC95, SALCARE™ SC96, FAOCARE™ ET100, FLOCARE™ ET58, HISPAGEL™, NOVEMER™ EC1, ARISTOFLEX™ AVC, ARISTOFLEX™ HBM, RAPITHIX™ A60, RAPITHIX™ A100, COSMEDIA SP et STABILEZE™ 06 ;
- les hydrocolloïdes d'origine végétale ou biosynthétique, par exemple la gomme de xanthane, la gomme de karaya, les carraghénates, les alginates, les galactomannanes ;
- les silicates ; la cellulose et ses dérivés ; l'amidon et ses dérivés hydrophiles ; les polyuréthanes.

Parmi les cires utilisables dans le cadre de la présente invention, on peut citer par exemple la cire d'abeille ; la cire de camauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante. Parmi les émulsionnants que l'on peut associer à la composition objet de l'invention, on peut citer par exemple :
- les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120.
- Les esters gras alkoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141.
- Les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.
- Les acides gras, les acides gras éthoxylés, les esters d'acide gras et de sorbitol, les esters d'acide gras et de mannitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435, 2 804 432, 2 830 774, 2 830 445, les associations de tensioactifs émulsionnants choisis parmi les alkylpolyglycosides, les associations d'alkylpolyglycosides et d'alcools gras, les esters de polyglycérol ou de polyglycol ou de polyols tels que les polyhydroxystéarates de polyglycol ou de polyglycérol mis en oeuvre dans les demandes de brevets français 2 852 257, 2 858 554, 2 820 316 et 2 852 258.

Comme exemples d'agents opacifiants et/ou nacrants que l'on peut associer à la composition objet de la présente invention, on peut citer les palmitates ou les stéarates ou les hydroxystéarates de sodium ou de magnésium, les monostéarates ou distéarates d'éthylène ou de polyéthylène glycol, les alcools copolymère commercialisé sous l'appellation MONTOPOL™ OP1 par la société SEPPIC.

Comme exemples de principes actifs que l'on peut associer à la composition objet de l'invention, afin d'en potentialiser par synergie ses propriétés, on peut citer : les composés ayant une action éclaircissante ou dépigmentante tels que par exemple l'arbutine, l'acide kojique, l'hydroquinone, l'acide ellagique, la vitamine C ou les dérivés de la Vitamine C comme par exemple le magnésium ascorbyl phosphate, les extraits de polyphénols, les extraits de raisin, les extraits de pin, les extraits de vin, les extraits d'olives, les extraits de marc, les extraits de jus de pomme, les dérivés d'acides aminés comme par exemple l'undécélénoyl phenylalanine commercialisé sous l'appellation SEPIWHITE MSH, les peptides ; les hydrolysâts totaux de protéines ; les hydrolysâts partiels de protéines ; les polyols (comme par exemple, la glycérine ou le butylène glycol) ; l'urée ; l'acide pyrrolidonecarboxylique ou les dérivés de cet acide ; l'acide glycyrrhétinique ; l'alpha-bisabolol ; les sucres ou les dérivés des sucres ; les polysaccharides ou leurs dérivés ; les hydroxyacides par exemple l'acide lactique ; les vitamines ou les dérivés de vitamines comme par exemple le Rétinol, la vitamine E et ses dérivés ; les sels minéraux ; les enzymes ; les co-enzymes comme par exemple le coenzyme Q10 ; les hormones ou "hormone like" comme par exemple le PHYTO-20 AGE™; les extraits de soja comme par exemple la Raffermine™ ; les extraits de blé comme par exemple la TensineTM ou la GliadineTM ; les extraits végétaux tels que les extraits riches en tanins, les extraits riches en isoflavones ou les extraits riches en terpènes ; les extraits d'algues d'eau douce ou d'algues marines ; les extraits de plantes marines ; les cires essentielles ; les extraits bactériens ; les lipides en général et plus particulièrement les lipides tels que les céramides ou les phospholipides ; les actifs ayant une activité antimicrobienne ou une action purifiante vis à vis des peaux grasses ; les actifs ayant une propriété énergisante ou stimulante comme le SEPITONIC™ M3 ou le Physiogényl™ ; le panthénol et ses dérivés comme le SEPICAP™ MP; les actifs anti-âge comme le SEPIVINOL™, le SEPIVITAL™, l'EXTRAMEL™ C, le MANOLIVA™ ; les actifs hydratants comme l'AQUAXYL™ ; les actifs " anti-photo vieillissement" ; les actifs présentant une action de tenseur ou de lissage immédiat sur la peau comme par exemple le SESAFLASH™ ; les actifs protecteurs de l'intégrité de la jonction dermo-épidermique comme le PHYTO-AGE™; les actifs augmentant la synthèse des composants de la matrice extracellulaire comme le SEPITON™ M3, l'AQUAXYL™ ; les actifs ayant une activité amincissante, raffermissante ou drainante comme par exemple la caféine, les dérivés de la caféine, la théophylline, l'Adénosyl Mono Phosphate cyclique (AMPc), l'ADIPOLESS™, le thé vert, la sauge, le ginko biloba, le lierre, le marron d'inde, le bambou, le ruscus, le petit houx, la centella asiatica, la bruyère, l'ulmaine, le fucus, le romarin, la saule, les extraits de panais, les extraits de potentille ; des actifs créant une sensation de « chauffe » sur la peau tels que les activateurs de la microcirculation cutanée comme exemple les nicotinates ; les actifs créant une sensation de « fraîcheur » sur la peau comme par exemple le menthol et ses dérivés ; les actifs présentant une action vis à vis des cellules souches ; les actifs présentant une action sur l'épiderme, le derme, l'hypoderme et les annexes cutanées (poils, ongles, glandes sébacées, pores...) ; les actifs présentant une action vis-à-vis de la flore cutanée.

Comme filtre solaire que l'on peut incorporer dans la composition selon l'invention, on peut citer tous ceux figurant dans la directive cosmétique 76/768/CEE modifiée annexe VII.

Lorsque la formulation cosmétique objet de l'invention est sous forme de gel crème, ladite formulation comprend : une résine telle que définie précédemment, un épaississant, une phase huileuse, un lipoaminoacide actif et de l'eau. Lorsque la formulation cosmétique objet de l'invention est sous forme d'émulsion, ladite formulation comprend : une résine telle que définie précédemment, un épaississant, une phase huileuse, un lipoaminoacide actif, au moins un tensioactif et de l'eau.

Toutes les formulations de la présente invention, sont stables lorsque leur pH est compris entre 5 et 7. En outre, lorsque la quantité de résine ne dépasse pas 1% de la masse totale, il n'y a pas de problème d'odeur ni de viscosité.

L'avantage de la solution de la présente invention est que cette technologie de piégeage de composés de formule (I) sur des résines échangeuses d'ions permet de présenter les molécules de composés de formule (I), se trouvant initialement sous la forme de liquides et/ou de pâtes, sous une forme de poudre fine, non collante et très facile à manipuler. Du fait des granulométries sélectionnées des résines de diamètre inférieur ou égal à 120 micromètres, le consommateur ne percevra pas la présence de particules solides dans la formulation cosmétique comprenant les composés de formule (I) piégés sur la résine échangeuse d'ions. Il y a donc un avantage galénique indéniable puisqu'une fois immobilisée sur une résine échangeuse d'ions, la molécule active pourra être incorporée dans une émulsion, une crème, un gel, une pâte ou même des poudres. Ceci sera possible quelle que soit la forme originale de la molécule. Il suffira d'arriver à introduire la molécule active sous forme liquide, soit en la diluant, soit en la fondant dans un liquide quelconque, qu'il soit aqueux, alcoolique ou hydrotrope.

Le mécanisme d'immobilisation de la molécule sur la résine échangeuse d'ions, de même que le mécanisme de sa libération sont contrôlés par des équilibres ioniques de réactions chimiques. On peut donc contrôler la libération des molécules actives en faisant varier la nature de la résine support ou la manière dont se fait la charge de cette résine. Cela est très difficile avec certaines formes de protection comme l'absorption sur support et totalement impossible avec des technologies comme la coacervation.

La taille des particules d'une résine échangeuse d'ions est très supérieure à la taille des pores de la peau. Donc le support polymérique qui transporte les molécules actives ne peut pas pénétrer sous la peau. C'est une assurance sur l'innocuité de la technologie pour l'utilisateur.

Les molécules concernées dans la présente invention sont entre autres, des molécules anti-rides, amincissantes, propigmentantes, dépigmentantes, éclaircissantes, émollients, apaisantes, hydratantes, asti-radicalaires, anti-acnéiques et assouplissantes de la peau.

Le procédé objet de la présente invention consiste à mettre en contact la résine choisie avec une solution aqueuse, alcoolique ou en solvant de la molécule que l'on veut piéger.

Cette mise en contact peut se faire par barbotage de la résine dans la solution à charger, puis filtration et rinçage pour récupérer la résine chargée. Mais on préférera un chargement de la résine par percolation, dans lequel un lit de résines va être traversé par la solution à charger.

En jouant sur la concentration de la solution de matière active, le temps de mise en contact et la nature de la résine, on pourra ensuite faire varier la vitesse de libération de la molécule active lorsque le résinate (ou la composition à base de résine et de composés de formule (I)) sera mise en contact avec la peau.

Cette libération se fera par échange d'ions entre la résine chargée et soit les cations (Na⁺, K⁺, Ca⁺, H⁺), soit les anions (Cl⁻, OH⁻) présents à la surface de la peau ou dans la sudation.

On peut également contrôler la vitesse de libération des molécules actives en mélangeant dans la formulation cosmétique considérée des résines chargées et des résines non chargées. Dans ce cas, la libération de la molécule active et son action à la surface de la peau sera en compétition avec le piégeage sur une résine non chargée. Donc la libération totale des molécules actives sera décrue et prolongée.

### Exemples

### 1) Préparation d'une composition (A) à base de N-(ω-undecylenoyl) phenylalanine et d'une résine échangeuse d'ions

Le N-(ω-undecylenoyl) phenylalanine, est un lipoaminoacide de formule brute C₂₀H₂₂NO₂, commercialisée par la société SEPPIC sous le nom de marque SEPIWHITE™ MSH, et possédant un poids moléculaire de 331 g / mol.

La capacité théorique de la résine échangeuse d'ions (Duolite AP 143 / 1073) est de 1,4 grammes de N-(ω-undecylenoyl) phenylalanine par grammes de résine. Une solution de 10 litres comprenant une proportion massique de 1% de N-(ω-undecylenoyl) phenylalanine salifiée sous forme de sel de sodium est tamponée à un pH de 7,5 et est agitée pendant deux heures en présence de 50 grammes de Duolite AP 143/1073 à l'aide d'un agitateur mécanique dans un réacteur équipé d'une double enveloppe permettant la circulation d'un fluide caloporteur.

Le mélange résultant est alors filtré sur un support de cellulose dont le diamètre des pores est de 25 micromètres. Le solide retenu sur un tel support filtrant est ensuite lavé par l'addition successive de deux solutions de 2 litres d'eau désionisée.

Le N-(ω-undecylenoyl) phenylalanine non greffé sur la résine et compris dans le filtrat précédemment est dosé par la mise en oeuvre d'une méthode HPLC (High Performance Liquid Chormatography).

Le dosage de N-(ω-undecylenoyl) phenylalanine dans le filtrat, par la mise en oeuvre d'une méthode HPLC (High Performance Liquid Chormatography), indique une quantité de 35 grammes de N-(ω-undecylenoyl) phenylalanine, signifiant ainsi que 65 grammes de N-(ω-undecylenoyl) phenylalanine ont été retenus sur la résine échangeuse d'ions, soit pour une quantité théorique de résine de 70 grammes et pour une quantité initiale de 100 grammes N-(ω-undecylenoyl) phenylalanine comprise dans la solution de 10 litres, un taux de greffage massique de 92% du N-(ω-undecylenoyl) phenylalanine sur la résine.

Après séchage, on obtient 109,3 grammes d'une composition (A) comprenant 56,5% massique de N-(ω-undecylenoyl) phenylalanine.

### 2) Préparation d'un gel crème et d'une émulsion comprenant la composition (A)

Un gel-crème et une émulsion ont été réalisés à différents pourcentages de Duolite® AP143/1073, à 2 pH différents.

### 2.1 Préparation d'un gel-crème:

a) On prépare une série de gel-crèmes, dont les compositions massiques en ingrédients sont indiquées dans le tableau 1 ci-dessous :

**Tableau 1 compositions des gel-crèmes GC1 à GC4**

| | GC 1 | GC 2 | GC 3 | GC 4 |
|---|---|---|---|---|
| **Phase huileuse:** | 3% | 3% | 3% | 3% |
| Triglycéride C8-C10 | | | | |
| ***Polymèrepolyélectrolyte*** | 3% | 3% | 3% | 3% |
| SEPIPLUS 400⁽¹⁾ | | | | |
| ***Phase aqueuse*** | | | | |
| - Eau | Qs 100% | Qs 100% | Qs 100% | Qs 100% |
| - Triéthanolamine à 99% | pH = 5 | Qs pH = 5 | Qs pH = 5 | Qs pH = 5 |
| - Sepicide ™ HB ⁽²⁾ | 1 % | 1 % | 1 % | 1% |
| Composition (A) | 0,5% | 0,8% | 1,0% | 0% |
| SEPIWHITE™MSH⁽³⁾ | 0% | 0% | 0% | 0,28% |

| | | | | |
|---|---|---|---|---|
| (1) SEPIPLUS™ 400 est un latex inverse auto-inversible de copolymères tel que ceux décrits dans la publication internationale WO 2005/040230 (Dénomination INCl : Polyacrylate-13 & Polyisobutene & Polysorbate 20), commercialisé par la société SEPPIC. (2) SEPICIDE™ HB est un mélange de phénoxyéthanol, de méthylparaben, d'éthylparaben, de propylparaben et de butylparaben, est un agent conservateur commercialisé par la société SEPPIC. (3) SEPIWHITETMMSH est le N-(w-undécylènoyl) phénylalanine commercialisé par la société SEPPIC comme agent éclaircissant de la peau. | | | | |

Les gel-crèmes, dont la composition est indiquée dans le tableau 1 ci-dessus, sont préparés en mettant en oeuvre le procédé suivant :
- étape a) : le polymère polyélectrolyte est dispersé à température ambiante dans l'eau prélablement introduite dans un bêcher de 2 litres et soumis à une agitation mécanique pendant une durée de 15 minutes, à l'aide d'un agitateur muni d'un mobile de type « ancre », à une vitesse de 80 tours/minute, de façon à former un gel homogène.
- étape b) : la phase huileuse, la composition (A) selon l'invention ou le SEPIWHITE™MSH, et l'agent conservateur SEPICIDE™ HB, sont ensuite ajoutés sur le gel formé lors de l'étape a), et le mélange résultant est soumis à une agitation par l'intermédiaire d'un émulseur de type rotor-stator, commercialisé par la société SILVERSON, pendant une durée de 5 minutes à une vitesse de 3000 tours/minute à une température de 25°C. Le pH est ensuite ajusté à une valeur de 5 par ajout de triéthanolamine à 99%.

### b) Evaluation des propriétés des gel-crèmes préparés.

La quantité des gel-crèmes, tels que préparés précédemment, est ensuite divisée en deux portions égales dans des bêchers différents, qui sont ensuite placés dans une enceinte climatique à température régulée à 45°C. L'expérimentateur examine alors l'aspect des gel-crèmes présents dans chaque enceinte climatique à des durées différentes. Les observations notées sont comprises dans le tableau ci-dessous :

**Tableau 2 évolution de l'aspect des gels-crèmes GCI à GC4 au stockage à 20°C et à 45°C.**

| | GC 1 | GC 2 | GC 3 | GC 4 |
|---|---|---|---|---|
| Aspect après 7 jours à 20°C | Homogène | Homogène | Homogène | Hétérogène, présence de grains |
| Aspect après 3 mois à 20°C | Homogène | Homogène | Homogène | Hétérogène, présence de grains |
| Aspect après 3 mois à 45°C | Homogène | Homogène | Homogène | Hétérogène, présence de grains |

Les résultats obtenus dans le tableau 2 ci-dessus montrent que la composition (A) selon l'invention, comprenant 56,5% massique de N-(ω-undecylenoyl) phenylalanine fixé sur la résine échangeuse d'ions Duolite AP 143 / 11073, permet de préparer des gel-crèmes GC1, GC2 et GC3, comprenant respectivement 0,28% massique de N-(ω-undecylenoyl) phenylalanine, 0,45% massique de N-(ω-undecylenoyl) phenylalanine et 0,56% massique de N-(ω-undecylenoyl) phenylalanine, qui sont homogènes après stockage à 20°C et à 45°C pendant une durée minimale de 3 mois, alors que le gel-crème GC4 comprenant 0,28% massique de N-(ω-undecylenoyl) phenylalanine, préparé dans les mêmes conditions et selon le même procédé, montre un aspect hétérogène après une durée de stockage de 7 jours à 20°C, se caractérisant par la présence de grains. Les gel-crèmes GC1, GC 2 et GC 3 procurent également une sensation de douceur persistante au consommateur lorsqu'ils sont appliqués sur la peau.

### 2.2 Préparation d'une émulsion huile-dans-eau :

a) On prépare une série d'émulsions huile-dans-eau, dont les compositions massiques en ingrédients sont indiquées dans le tableau 3 ci-dessous :

**Tableau 3 : compositions des émulsions huile-dans-eau EMI à EM4**

| | EM 1 | EM 2 | EM 3 | EM4 |
|---|---|---|---|---|
| **Phase huileuse:** | 10% | 10% | 10% | 10% |
| Triglycéride C8-C10 | | | | |
| ***Polymèrepolyélectrolyte*** | | | | |
| SEPIPLUS 400⁽¹⁾ | 0,7% | 0,7% | 0,7% | 0,7% |
| ***Systèmeémulsionnant*** | | | | |
| MONTANOV 68⁽⁴⁾ | 2,5% | 2,5% | 2,5% | 2,5% |
| MONTANOV 202⁽⁵⁾ | 2,5% | 2,5% | 2,5% | 2,5% |
| ***Phase aqueuse*** | | | | |
| - Eau | Qs 100% | Qs 100% | Qs 100% | Qs 100% |
| - Triéthanolamine à 99% | QspHr= 5 | Qs pH = 5 | Qs pH = 5 | QspH= 5 |
| - Sepicidem FIB⁽²⁾ | 1% | 1% | 1% | 1% |
| Composition A | 0,5% | 0,8% | 1,0% | 0% |
| SEPIWHITE™MSH⁽³⁾ | 0% | 0% | 0% | 0,28% |

| | | | | |
|---|---|---|---|---|
| (4) MONTANOV™ 68 (alcool cétéarylique et cétéraryl glucoside), est une composition auto-émulsionnable commercialisée par la société SEPPIC. (5) MONTANOV™ 202 (alcool arachydilique, alcool béhénique et arachidyl glucoside), est une composition auto-émulsionnable telle que celles décrites dans EP 0977626, commercialisée par la société SEPPIC. | | | | |

Les émulsions, dont la composition est indiquée dans le tableau 3 ci-dessus, sont préparées en mettant en oeuvre dans le procédé suivant :
- étape a) la phase huileuse et le système émulsionnant sont introduits successivement dans un bêcher de 2 litres, à une température de 75°C et sont soumis à une agitation mécanique pendant une durée de 15 minutes, à l'aide d'un agitateur muni d'un mobile de type « ancre », à une vitesse de 80 tours/minute à une température de 75°C, de façon à former un mélange homogène.
- étape b) : le polymère polyélectrolyte est ajouté sur le mélange obtenu à l'issue de l'étape a) à une température de 75°C ainsi que l'eau. Le mélange résultant est soumis à une agitation par l'intermédiaire d'un émulseur de type rotor-stator, commercialisé par la société SILVERSON, pendant une durée de 4 minutes à une vitesse de 3000 tours/minute à une température de 75°C.
- étape c) : Le mélange obtenu à l'issue de l'étape b) est refroidi pendant 10 minutes à une température de 30°C, et maintenu sous une agitation pendant la durée de refroidissement par l'intermédiaire d'un agitateur mécanique muni d'un mobile de type « ancre », à une vitesse de 80 tours/minute.
- étape d) : la composition (A) selon l'invention ou le SEPIWHITE™MSH, et l'agent conservateur SEPICIDE™ HB, sont ensuite ajoutés sur le mélange issu de l'étape c) à une température de 30°C. Le pH est ensuite ajusté à une valeur de 5 par ajout de triéthanolamine à 99%.

### b) Evaluation des propriétés des émulsions préparées :

La quantité de chaque émulsion est ensuite divisée en deux portions égales dans des bêchers différents, qui sont ensuite placés dans une enceinte climatique à température régulée à 20°C et dans une enceinte climatique à température régulée à 45°C. L'expérimentateur examine alors l'aspect des émulsions présentes dans chaque enceinte climatique à des durées différentes. Les observations notées sont comprises dans le tableau 4 ci-dessous :

**Tableau 4 évolution de l'aspect des émulsions huile-dans-eau EMI a EM4 au stockage à 20°C et à 45°C.**

| | EM 1 | EM 2 | EM 3 | EM 4 |
|---|---|---|---|---|
| Aspect après 48 heures à 20°C | Homogène | Homogène | Homogène | Hétérogène, présence de grains |
| Aspect après 3 mois à 20°C | Homogène | Homogène | Homogène | Hétérogène, Présence de grains |
| Aspect après 3 mois à 45°C | Homogène | Homogène | Homogène | Hétérogène, présence de grains |

Les résultats obtenus dans le tableau 4 ci-dessus montrent que la composition (A) selon l'invention, comprenant 56,5% massique de N-(ω-undecylenoyl) phenylalanine fixé sur la résine échangeuse d'ions Duolite AP 143 / 1073, permet de préparer des émulsions huile-dans-eau EM1, EM2 et EM3, comprenant respectivement 0,28% massique de N-(ω-undecylenoyl) phenylalanine, 0,45% massique de N-(ω-undecylenoyl) phenylalanine et 0,56% massique de N-(ω-undecylenoyl) phenylalanine, qui sont homogènes après stockage à 20°C et à 45°C pendant une durée minimale de 3 mois, alors que l'émulsion EM4 comprenant 0,28% massique de N-(ω-undecylenoyl) phenylalanine, préparée dans les mêmes conditions et selon le même procédé, montre un aspect hétérogène après une durée de stockage de 48 heures à 20°C, se caractérisant par la présence de grains. Les émulsions EM1, EM2 et EM3 procurent également une sensation de douceur persistante au consommateur lorsqu'elles sont appliquées sur la peau.

### Exemples de formulation cosmétiques

### Exemple 3 : Emulsion-Soin éclaircissant pour peau mature

| | |
|---|---|
| MONTANOV™ 202 | 02, 00 % |
| MONTANOV™ 68 | 02,00 % |
| Caprylic capric triglycérides | 10,00 % |
| Squalane | 10,00 c/o |
| Eau | QSP 100 % |
| Composition A | 0,54 % |
| SEPIGEL™ 305 | 00,70 % |
| Magnésium ascorbyl phosphate | 02,00 % |
| SEPICIDE™ HB | 00,30 % |
| SEPICIDE™ Cl | 00,20 % |
| Parfum | 00,50 % |

### Exemple : 4 Emulsion-Soin raffermissant éclaircissant

| | |
|---|---|
| MONTANOV™ 202 | 03, 00 % |
| Soude à 24 % | 00,06 % |
| Ethyl hexyl méthoxycinnamate | 06,00 % |
| LANOL™ 1688 | 08,00 % |
| Benzophénone-3 | 04,00 % |
| Eau | QSP 100 % |
| Composition A | 0,87 % |
| SIMULGEL™ NS | 00,50 % |
| Sepilift™ DPHP | 00,50 % |
| Diméthicone | 02,00 % |
| Cyclométhicone | 02,00 % |
| Arbutine | 0.3 % |
| SEPICIDE™ HB | 00,30 % |
| SEPICIDE™ Cl | 00,20 % |
| Parfum | 00,10 % |

### Exemple 5 : Gel-crème éclaircissant aux alphahydroxyacides

| | |
|---|---|
| Hydroxyéthylcellulose | 00,80 % |
| Ethylhexyl octanoate | 05,00 % |
| Sodium lactate à 60 % | 14,00 % |
| Eau | QSP 100 % |
| Composition A | 1,0 % |
| SEPIGEL™ 305 | 04,20 % |
| SEPICIDE™ HB | 02,00 % |
| SEPICIDE™ Ci | 03,00 % |
| Parfum | 00,10 % |

### Exemple 6 : Emulsion- Soin éclaircissant

| | |
|---|---|
| MONTANOV™ L | 01,00 % |
| Cetyl alcool | 02,00 % |
| Isodecyl neopentanoate | 12,00 % |
| Cetearyl octanoate | 10,00 % |
| Glycérine | 03,00 % |
| Eau | QSP 100 % |
| Composition A | 0,54 % |
| SIMULGEL™ EG | 02,00 % |
| Acide kojique | 01,00 % |
| SEPICIDE™ HB | 00,30 % |
| SEPICIDE™ Cl | 00,20 % |
| Parfum | 00,10 % |

### Exemple 7 : Lotion éclaircissante

| | |
|---|---|
| ORAMIX™ CG110 | 05,00 % |
| KATHON™ CG | 00,08 % |
| Eau | QSP 100 % |
| Composition A | 0,54 % |
| Parfum | 00,10% |

Cette lotion peut être vendue en flacons ou imprégnée sur des lingettes.

Les définitions des produits commerciaux utilisés dans les exemples sont les suivantes :
SEPILIFT™ DHP (Dénomination INCl : Dipalmitoyl hydroxyproline), commercialisé par la société SEPPIC.
SEPICIDE™ HB est un mélange conservateur comprenant du Phenoxyethanol, du méthylparaben, de l'éthylparaben, du propylparaben et du butyl paraben, commercialisé par la société SEPPIC.
SEPICIDE™ CI est de l'imidazolidinyl urée, commercialisé par la société SEPPIC.
SEPICALM™ VG (Dénomination INCl : sodium palmitoyl proline et extrait de fleur de nénuphar), commercialisé par la société SEPPIC.
KATHON™ CG (Dénommination INCl: méthyl isothiazolinone / Méthyl chloroisothiazolinone).
SIMULGEL™ EG est un latex inverse de copolymère (dénomination INCl: Sodium acrylate/Sodium acryloyldimethyl taurate copolymer et Isohexadecane et Polysorbate 80) commercialisé par la société SEPPIC.
SIMULGEL™ NS est un latex inverse de copolymère (dénomination INCl : hydroxyethylacrylate/Sodium acryloyldimethyl taurate copolymer et squalane et Polysorbate 60) commercialisé par la société SEPPIC.
LANOL™ 1688 est du cétéaryl ethylhexanoatel commercialisé par la société SEPPIC.
SEPIGEL™ 305 est un latex inverse de polymère (dénomination INCl polyacrylamide et isoparaffine en C13-C14 et laureth 7).
MONTANOV™ L est un agent émulsionnant à base d'alcool en C14-C22 et d'alkyl polyglucoside en C12-C20.
MONTANOV™ 68 est un agent émulsionnant à base d'alcool cétéarylique et cétéaryl polyglucoside.
MONTANOV™ 202 est un agent émulsionnant à base d'alcool arachidylique d'alcool béhènylique et d'arachidyl polyglucoside.

Remarque : selon la capacité d'échange de la résine vis à vis des lipomaminoacides, la quantité de résine à utiliser est de l'ordre de 0,5% pour avoir 1% de lipoaminoacide dans la formule finale, d'où les concentrations testées dans les schémas de formule ci-dessus.

## Revendications

1. Composition de résines échangeuses d'ions à base de copolymère styrène - divinylbenzène ou de copolymère acrylique - divinylbenzène chargées avec au moins un lipoaminoacide de formule (I) : 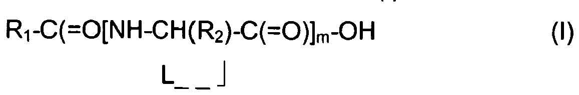 dans laquelle R₁ représente la chaîne caractérisante d'un acide gras, saturé ou insaturé, linéaire ou ramifié, comportant de 7 à 21 atomes de carbone, R₂ représente la chaîne caractérisante d'un acide aminé et m est compris entre 1 et 50, ou d'un mélange desdits composés de formule (I).

2. Composition selon la revendication 1, **caractérisée en ce que** ladite résine contient au moins une fonction ammonium quaternaire.

3. Composition selon la revendication 1 ou 2, **caractérisée en ce que** ledit lipoaminoacide est choisi parmi : le N-(ω-undecylenoyl) phenylalanine, l'octanoyl glycine, l'undecylenoyl glycine, le palmitoyl proline, le dipalmitoylhydroxyproline, le cocoylalanine, le palmitoylglycine.

4. Formulation cosmétique comprenant la composition telle que définie à l'une des revendications 1 à 3, **caractérisée en ce que** le pH de ladite formulation est compris entre 5 et 7 et la proportion massique de ladite composition est inférieure à 1,5% de la masse totale.

5. Formulation selon la revendication 4, **caractérisée en ce que** la proportion massique de la composition telle que définie à l'une des revendications 1 à 3 est comprise entre 0,5% et 0,8% de la masse totale.

6. Formulation selon l'une des revendications 4 et 5 **caractérisée en ce qu'**elle se présente sous la forme d'un gel crème, d'une émulsion, d'une poudre, d'une dispersion aqueuse, d'une dispersion huileuse.

7. Utilisation d'une formulation telle que définie à l'une des revendications 4 à 6 pour une application cosmétique sur la peau humaine.

8. Utilisation d'une formulation telle que définie à l'une des revendications 4 à 6, comme antiride **caractérisée en ce que** le lipoaminoacide employé est choisi parmi : le dipalmitoylhydroxy proline, le cocoylalanine, le palmitoylglycine.

9. Utilisation d'une formulation telle que définie à l'une des revendications 4 à 6, comme agent d'éclaircissement de la peau ou agent amincissant **caractérisée en ce que** le lipoaminoacide employé est choisi parmi : le N-(ω-undecylenoyl) phenylalanine, le palmitoyl proline.

10. Procédé de préparation d'une composition telle que définie à l'une des revendications 1 à 3, comprenant l'étape
a) mise en contact d'une résine échangeuse d'ions à base de copolymère styrène - divinylbenzène ou styrène - acrylique contenant au moins un ammonium quaternaire avec une solution de lipoaminoacide de formule (I).

11. Procédé selon la revendication 10, **caractérisé en ce que** l'étape a) est une étape de percolation où un lit de ladite résine est traversé par ladite solution de lipoaminoacide à charger.

## Patentansprüche

1. Zusammensetzung aus Ionenaustauschharzen auf Basis von Styrol-Divinylbenzol-Copolymer oder Acryl-Divinylbenzol-Copolymer, die angereichert sind mit mindestens einer Lipoaminosäure der Formel (I): 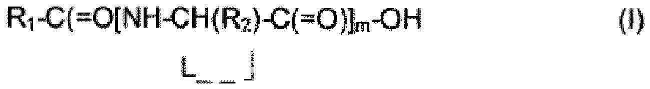 worin R₁ die charakterisierende Kette einer gesättigten oder ungesättigten geradkettigen oder verzweigten Fettsäure mit 7 bis 21 Kohlenstoffatomen bedeutet, R₂ die charakterisierende Kette einer Aminosäure bedeutet und m zwischen 1 und 50 beträgt, oder eines Gemischs der Verbindungen der Formel (I).

2. Zusammensetzung nach Anspruch 1, **dadurch gekennzeichnet, dass** das Harz mindestens eine quaternäre Ammoniumfunktion enthält.

3. Zusammensetzung nach Anspruch 1 oder 2, **dadurch gekennzeichnet, dass** die Lipoaminosäure ausgewählt ist aus: N-(ω-Undecylenoyl)-phenylalanin,Octanoyl-glycin, Undecylenoyl-glycin, Palmitoyl-prolin, Dipalmitoylhydroxy-prolin, Cocoylalanin, Palmitoyl-glycin.

4. Kosmetische Formulierung, enthaltend die Zusammensetzung nach einem der Ansprüche 1 bis 3, **dadurch gekennzeichnet, dass** der pH-Wert der Formulierung zwischen 5 und 7 beträgt und der Masseanteil der Zusammensetzung kleiner als 1,5 % der Gesamtmasse ist.

5. Formulierung nach Anspruch 4, **dadurch gekennzeichnet, dass** der Masseanteil der Zusammensetzung nach einem der Ansprüche 1 bis 3 zwischen 0,5 % und 0,8 % der Gesamtmasse beträgt.

6. Formulierung nach einem der Ansprüche 4 und 5, **dadurch gekennzeichnet, dass** sie die Form einer Gelcreme, einer Emulsion, eines Puders, einer wässrigen Dispersion, einer öligen Dispersion aufweist.

7. Verwendung einer Formulierung nach einem der Ansprüche 4 bis 6 für eine kosmetische Anwendung auf der menschlichen Haut.

8. Verwendung einer Formulierung nach einem der Ansprüche 4 bis 6 als Antifaltenmittel, **dadurch gekennzeichnet, dass** die verwendete Lipoaminosäure ausgewählt ist aus: Dipalmitoylhydroxy-prolin, Cocoylalanin, Palmitoyl-glycin.

9. Verwendung einer Formulierung nach einem der Ansprüche 4 bis 6 als Mittel zum Aufhellen der Haut oder Schlankheitsmittel, **dadurch gekennzeichnet, dass** die verwendete Lipoaminosäure ausgewählt ist aus: N-(ω-Undecylenoyl)-phenylalanin, Palmitoyl-glycin.

10. Verfahren zur Herstellung einer Zusammensetzung nach einem der Ansprüche 1 bis 3, umfassend den folgenden Schritt:
a) Inkontaktbringen eines Ionenaustauschharzes auf Basis von Styrol-Divinylbenzol-Copolymer oder Acryl-Divinylbenzol-Copolymer, das mindestens ein quaternäres Ammoniumharz enthält, mit einer Lipoaminosäure-Lösung der Formel (I).

11. Verfahren nach Anspruch 10, **dadurch gekennzeichnet, dass** Schritt a) ein Perkolationsschritt ist, wo die anzureichernde Lipoaminosäure-Lösung durch ein Bett aus dem Harz geführt wird.

## Claims

1. Composition of styrene-divinylbenzene copolymer-based or acrylic-divinylbenzene copolymer-based ion-exchange resins loaded with at least one lipoamino acid of formula (I): 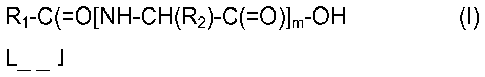 in which R₁ represents the characterising chain of a linear or branched, saturated or unsaturated fatty acid comprising from 7 to 21 carbon atoms, R₂ represents the characterising chain of an amino acid and m is between 1 and 50, or of a mixture of said compounds of formula (I),

2. Composition according to claim 1, **characterised in that** said resin contains at least one quaternary ammonium function.

3. Composition according to either claim 1 or claim 2, **characterised in that** said lipoamino acid is selected from: N-(ω-undecylenoyl) phenylalanine, octanoylglycine, undecylenoyl glycine, palmitoyl proline, dipalmitoyl hydroxyproline, cocoylalanine and palmitoylglycine.

4. Cosmetic formulation comprising the composition according to any of claims 1 to 3, **characterised in that** said formulation has a pH between 5 and 7, and the proportion by weight of said composition is less than 1.5 % of the total weight.

5. Formulation according to claim 4, **characterised in that** the proportion by weight of the composition according to any of claims 1 to 3 is between 0.5 % and 0.8 % of the total weight.

6. Formulation according to either claim 4 or claim 5, **characterised in that** it is in the form of a gel cream, an emulsion, a powder, an aqueous dispersion or an oily dispersion.

7. Use of a formulation according to any of claims 4 to 6 for cosmetic application on human skin.

8. Use of a formulation according to any of claims 4 to 6 as an anti-wrinkle agent, **characterised in that** the lipoamino acid used is selected from: dipalmitoyl hydroxyproline, cocoylalanine and palmitoylglycine.

9. Use of a formulation according to any of claims 4 to 6 as a skin-lightening agent or slimming agent, **characterised in that** the lipoamino acid used is selected from: N-(ω-undecylenoyl) phenylalanine and palmitoyl proline.

10. Method for preparing a composition according to any of claims 1 to 3, comprising the step of:
a) bringing a styrene-divinylbenzene copolymer-based or a styrene-acrylic copolymer-based ion-exchange resin containing at least one quaternary ammonium into contact with a solution of lipoamino acid of formula (I).

11. Method according to claim 10, **characterised in that** step a) is a percolation step in which said solution of lipoamino acid to be loaded passes through a bed of said resin.
